# EUROPÄISCHE PATENTSCHRIFT

(11) **EP 1 328 306 B1**
(45) Veröffentlichungstag und Bekanntmachung des Hinweises auf die Patenterteilung: **23.11.2005**
(21) Anmeldenummer: 01982122.2
(22) Anmeldetag: 19.09.2001
(51) Int. Cl.: A61M 1/14

(54) **WERKSTOFF ZUR HERSTELLUNG VON ERZEUGNISSEN MIT FIBRINOLYTISCHEN UND/ODER ANTIBAKTERIELLEN EIGENSCHAFTEN**
MATERIAL FOR PRODUCING PRODUCTS HAVING FIBRINOLYTIC AND/OR ANTIBACTERIAL PROPERTIES
MATERIAL UTILISE POUR FABRIQUER DES PRODUITS AUX PROPRIETES FIBRINOLYTIQUES ET/OU ANTIBACTERIENNES

(30) Priorität: 10.10.2000 DE 10050317
(43) Veröffentlichungstag der Anmeldung: 23.07.2003
(73) Patentinhaber: Intech Thüringen GmbH, 99880 Waltershausen (DE)
(72) Erfinder: SCHUNK, Werner, 99867 Gotha (DE); BRUDER, Michael, 22147 Hamburg (DE); GIESSMANN, Konrad, 99867 Gotha (DE); KRAUSE, Karl-Heinz, 09123 Chemnitz (DE); MERKMANN, Gerhard, 99867 Gotha (DE)
(86) Internationale Anmeldenummer: PCT/DE2001/003587
(87) Internationale Veröffentlichungsnummer: WO 2002/030388

(56) Entgegenhaltungen:
- EP-A- 0 640 661
- DD-A- 264 009
- DD-A- 270 867
- DD-A- 278 492
- DD-A- 294 959
- US-A- 6 071 542

## Beschreibung

Die Erfindung betrifft einen Werkstoff zur Herstellung von Erzeugnissen mit fibrinolytischen und/oder antibakteriellen Eigenschaften, umfassend wenigstens eine körpergewebeverträgliche Matrix, in die ein Molekularsieb eingemischt ist, das mit
- wenigstens einem Wirkstoff mit fibrinolytischer oder antibakterieller Wirkung oder
- wenigstens einem ersten Wirkstoff mit fibrinolytischer Wirkung und einem zweiten Wirkstoff mit antibakterieller Wirkung
beladen ist, wobei das entsprechende Molekularsieb/Wirkstoff-Addukt bei Kontakt der Matrix mit dem Körpergewebe in Verbindung mit der wässrigen Körperflüssigkeit den/die Wirkstoff/e freigibt.

Werkstoffe (Biomaterialien) als Ersatz körperlichen Gewebes (z.B. Herzklappen) sind aus der modernen Chirurgie nicht mehr wegzudenken. Die im Körper verbleibenden Werkstoffe müssen in erster Linie biologisch inert sein, d.h. im menschlichen und tierischen Organismus verträglich und ohne Abwehrreaktion vom Körper für die Zeit des Verweilens im Organismus akzeptiert werden.

In Verbindung mit diesen Werkstoffen spielen Molekularsieb/Wirkstoff-Addukte eine große Rolle. Von Bedeutung sind dabei Wirkstoffe mit fibrinolytischer Wirkung (Fibrinolytika), beispielsweise Heparinoide, oder mit antibakterieller Wirkung, beispielsweise Antibiotika. Besonders vorteilhaft ist es, wenn beide Wirkstofftypen kombinativ eingesetzt werden. Hinsichtlich des diesbezüglichen Standes der Technik wird insbesondere auf die Druckschriften DD 294 959 A5, DD 295 649 A5, DE 196 81 282 T1 und DE 694 21 351 T2 verwiesen.

Aus dem Molekularsieb/Wirkstoff-Addukt wird der Wirkstoff desorptiv freigesetzt, indem das Wasser des Körpergewebes in die Matrix eindiffundiert. Nachfolgend wird der Wirkstoff gelöst und diffundiert dann zum Körpergewebe.

Bei den bisher bekannten Werkstoffen dieser Art trat entweder ein "Spritzeneffekt" ein, d.h. ein schneller Abbau des Wirkstoffes und somit eine wesentliche Verringerung der medikamentösen Depotwirkung, oder die Freisetzung des Wirkstoffes verlief unkontrolliert.

Im Rahmen einer Weiterentwicklung besteht die Aufgabe der Erfindung darin, einen Werkstoff zur Herstellung von Erzeugnissen mit fibrinolytischen und/oder antibakteriellen Eigenschaften bereitzustellen, bei dem ein kontinuierliches Wirkstoffangebot gewährleistet ist. Darüber hinaus soll bei geringerer Medikamentenbelastung kurze Behandlungszeiten erreicht werden, und zwar bei gleichzeitiger Erweiterung des medizinischen Anwendungsspektrums.

Zwecks Lösung dieser Aufgabe zeichnet sich nun der erfindungsgemäße Werkstoff nach dem Kennzeichen des Patentanspruches 1 dadurch aus, dass das Molekularsieb/Wirkstoff-Addukt zusätzlich Kristallwasser enthält, und zwar derart, dass in Bezug auf eine ausreichende Grundmolmenge (m) an Kristallwasser das Molekularsieb partiell dehydratisiert ist, wobei das Molekularsieb mit der reduzierten Molmenge (m') an Kristallwasser mit dem/den Wirkstoff/en beladen ist, so dass bei Kontakt der Matrix mit dem Körpergewebe eine Adsorption von Wasser unter Desorption des entsprechenden Wirkstoffes stattfindet, wobei der Kristallwassergehalt des Molekularsiebes zunimmt.

Zweckmäßige Gestaltungsvarianten des Werkstoffes sind in den Patentansprüchen 2 bis 16 genannt.

Darüber hinaus besteht die Aufgabe der Erfindung darin, ein Verfahren zur Herstellung des erfindungsgemäßen Werkstoffes bereitzustellen.

Gemäß Kennzeichen des Patentanspruches 17 zeichnet sich nun das Verfahren zur Herstellung des neuen Werkstoffes durch folgende Verfahrensschritte aus:
- das Molekularsieb mit einer ausreichenden Grundmolmenge (m) an Kristallwasser wird bei 300 bis 500°C, vorzugsweise bei 400 bis 450°C, mehrere Stunden, vorzugsweise 2 bis 6 Stunden, lang partiell dehydratisiert;
- anschließend wird das partiell dehydratisierte Molekularsieb mit der reduzierten Molmenge (m') an Kristallwasser mit dem/den Wirkstoff/en unter Bildung des entsprechenden Molekularsieb/Wirkstoff-Adduktes beladen;
- schließlich wird das Molekularsieb/Wirkstoff-Addukt in die Matrix eingemischt.

Die Dehydratisierung und/oder Beladung wird/werden dabei insbesondere in Gegenwart eines inerten Gases, beispielsweise von Stickstoff, durchgeführt. Die Dehydratisierung und/oder Beladung erfolgt/erfolgen ferner vorzugsweise unter Normaldruck. Die Beladung selbst kann beispielsweise mittels einer Kugelmühle vorgenommen werden.

Die Erfindung wird nun anhand von Ausführungsbeispielen unter Bezugnahme auf schematische Zeichnungen erläutert. Es zeigen:
- Fig. 1: den Adsorption/Desorptions-Mechanismus anhand von zwei Ausführungsbeispielen;
- Fig. 2: den Adsorption/Desorptions-Mechanismus anhand zweier Molekularsieb/Wirkstoff-Addukte, die einen unterschiedlichen Dehydratisierungsgrad aufweisen.

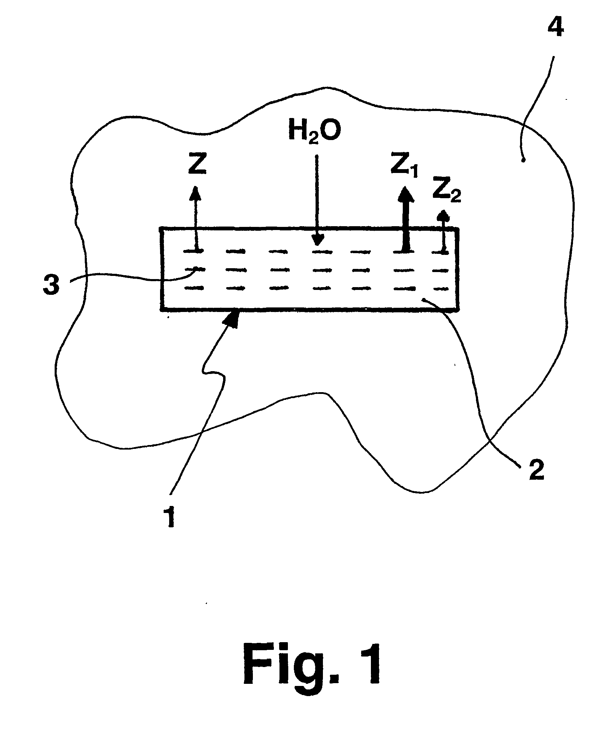

In Form eines einfachen Ausführungsbeispieles umfasst das medizinische Erzeugnis **1** gemäß Fig. 1 eine Matrix **2**, die dem spezifischen Anwendungsbereich entsprechend geformt ist.

In die Matrix **2** ist nun das Molekularsieb/Wirkstoff-Addukt **3** eingemischt. Als Molekularsieb wird insbesondere ein Natrium-Aluminium-Silikat der Formel

Na₈₆ [(AlO₂)₈₆ · (SiO₂)₁₀₆] · m(m')H₂O

verwendet, das im noch nicht dehydratisierten Zustand eine Grundmolmenge (m = 276) an Kristallwasser enthält. Im Rahmen der partiellen Dehydratisierung werden dabei mindestens 20 %, vorzugsweise 40 bis 70 %, Mole Wasser entzogen. Das partiell dehydratisierte Molekularsieb mit der reduzierten Molmenge (m'; z.B. m' = 200) ist nun mit einem Wirkstoff Z beladen.

Bei Kontakt der Matrix **2** mit dem Körpergewebe **4** wird nun diesem Wasser entzogen, das von dem Molekularsieb/Wirkstoff-Addukt 3 aufgenommen wird (Adsorption), wobei eine Desorption des Wirkstoffes Z mit fibrinolytischer oder antibakterieller Wirkung stattfindet. Dabei nimmt der Kristallwassergehalt des Molekularsiebes zu. Das Molekularsieb erhält also das Kristallwasser zurück, das man ihm im Rahmen der partiellen Dehydratisierung entzogen hat.

Die Tatsache, dass das Molekularsieb/Wirkstoff-Addukt **3** grundsätzlich Kristallwasser enthält, sorgt für ein entsprechendes Feuchtmilieu. Auf diese Weise wird die Desorption des Wirkstoffes Z mittels des Lösungs- bzw. Dispergierungsmittels Wasser erleichtert. Zusätzlich schafft das feuchte Milieu ideale Bedingungen für Wachstumsfaktoren der Zellen und dient zudem zur Immunabwehr.

Im Rahmen der gleichen Figur ist ein weiteres Ausführungsbeispiel dargestellt. Hiernach ist das Molekularsieb bei einem einheitlichen Dehydratisierungsgrad mit einem ersten Wirkstoff Z₁ mit fibrinolytischer Wirkung und einem zweiten Wirkstoff Z₂ mit antibakterieller Wirkung beladen, die sich durch einen unterschiedlichen Beladungsgrad auszeichnen, so dass das Molekularsieb/Wirkstoff-Addukt **3** bei Kontakt der Matrix **2** mit dem Körpergewebe **4** die Wirkstoffe Z₁ und Z₂ sequentiell freigibt, was durch die unterschiedlichen Pfeilstärken dargestellt ist. Die beiden Wirkstoffe haben dabei etwa das gleiche Molekulargewicht. Folgendes Zahlenbeispiel soll dies verdeutlichen. Das Molekularsieb weist einen Dehydratisierungsgrad von 70 % auf. Bei einem Gesamtbeladungsgrad von 60 % an den Wirkstoffen Z₁ und Z₂ entfallen auf Z₁ 40 % und Z₂ 20 %. Aufgrund der größeren Beladungsmenge wird der Wirkstoff Z₁ schneller abgegeben als der Wirkstoff Z₂, verbunden mit einer zeitlich versetzten Therapiewirkung.

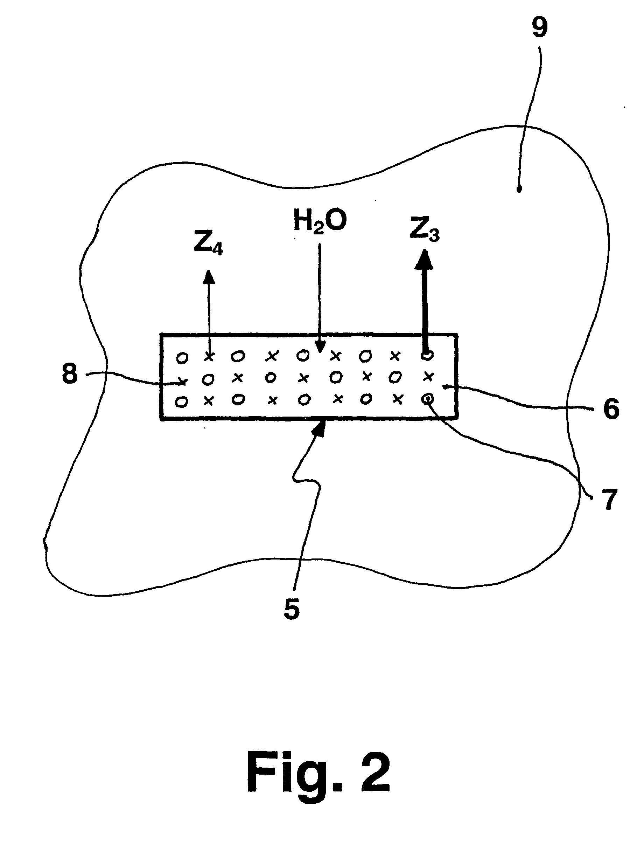

Eine beispielhafte medizinische Anwendung in diesem Sinne wäre die Kombination eines Fibrinolytikums (Z₁) mit einem Antibiotikum (Z₂), das auch für das Ausführungsbeispiel gemäß Fig. 2 herangezogen werden kann, das nun näher vorgestellt wird.

In die Matrix **6** sind ein erstes und zweites Molekularsieb **7** bzw. **8** eingemischt, die typengleich sind, sich jedoch durch einen unterschiedlichen Dehydratisierungsgrad auszeichnen, wobei folgendes Beispiel genannt sei:
Molekularsiebtyp: Na₈₆ [(AlO₂)₈₆ · (SiO₂)₁₀₆] · m(m')H₂O
Grundmolmenge (m) vor der partiellen Dehydratisierung: m = 276
reduzierte Molmenge (m')
   - Molekularsieb/Wirkstoff-Addukt **7** mit dem Wirkstoff Z₃ : m' = 100
   - Molekularsieb/Wirkstoff-Addukt **8** mit dem Wirkstoff Z₄ : m' = 200

Bei etwa gleichem Molekulargewicht der Wirkstoffe Z₃ (Fibrinolytikum) und Z₄ (Antibiotikum) ist dabei jeweils der Beladungsgrad etwa gleich.

Bei Kontakt der Matrix **6** mit dem Körpergewebe **9** werden die Wirkstoffe Z₃ und Z₄ sequentiell freigegeben, was wiederum durch die unterschiedlichen Pfeilstärken symbolisiert ist. Mit anderen Worten: Das Addukt **7** mit dem größeren Dehydratisierungsgrad wird begieriger Wasser aufnehmen als das Addukt **8**. Folge ist, dass der Wirkstoff Z₃ schneller abgegeben wird als der Wirkstoff Z₄, verbunden wiederum mit einer zeitlich versetzten Therapiewirkung.

Unabhängig von den Ausführungsbeispielen gelten für den Werkstoff zweckmäßigerweise folgende Parameter:
- Die Addukte **3**, **7** und **8** sind innerhalb der Matrix **2** und **6** im wesentlichen gleichmäßig verteilt.
- Die Addukte **3**, **7** und **8** weisen einen Anteil von 2 bis 30 Gew.-%, vorzugsweise 15 bis 20 Gew.-%, auf, und zwar bezogen auf die Gesamtmasse der Matrix **2** und **6**.
- Die Matrix **2** und **6** ist ein Polymerwerkstoff, insbesondere wiederum ein Elastomer, ein thermoplastisches Elastomer oder ein thermoplastischer Kunststoff. Wichtig in diesem Zusammenhang ist, dass diese Werkstoffe eine körpergewebeverträgliche Matrix bilden.

Der Werkstoff wird üblicherweise steril verpackt.

Die in der Beschreibung und in den Patentansprüchen erwähnten Aussagen zum Dehydratisierungsgrad und Beladungsgrad beziehen sich auf den Zustand nach Abschluss der partiellen Dehydratisierung bzw. der Beladung, d.h. ohne Stoffwechselaustausch von Wasser und Wirkstoff.

Der in den Fig. 1 und 2 dargestellte Stoffaustausch von Wasser und Wirkstoffen in eine Richtung hat nur schematischen Charakter. In Wirklichkeit findet dieser Stoffaustausch in allen Richtungen statt.

### Bezugszeichenliste

- **1**: Erzeugnis mit fibrinolytischen und/oder antibakteriellen Eigenschaften
- **2**: Matrix
- **3**: Molekularsieb/Wirkstoff-Addukt mit den Wirkstoffen Z, Z₁, Z₂
- **4**: Körpergewebe
- **5**: Erzeugnis mit fibrinolytischen und antibakteriellen Eigenschaften
- **6**: Matrix
- **7**: Molekularsieb/Wirkstoff-Addukt mit dem Wirkstoff Z₃
- **8**: Molekularsieb/Wirkstoff-Addukt mit dem Wirkstoff Z₄
- **9**: Körpergewebe

## Patentansprüche

1. Werkstoff zur Herstellung von Erzeugnissen (1, 5) mit fibrinolytischen und/oder antibakteriellen Eigenschaften, umfassend wenigstens eine körpergewebeverträgliche Matrix (2, 6), in die ein Molekularsieb eingemischt ist, das mit
- wenigstens einem Wirkstoff (Z) mit fibrinolytischer oder antibakterieller Wirkung oder
- wenigstens einem ersten Wirkstoff (Z₁, Z₃) mit fibrinolytischer Wirkung und einem zweiten Wirkstoff (Z₂, Z₄) mit antibakterieller Wirkung
beladen ist, wobei das entsprechende Molekularsieb/Wirkstoff-Addukt (3, 7, 8) bei Kontakt der Matrix (2, 6) mit dem Körpergewebe (4, 9) in Verbindung mit der wässrigen Körperflüssigkeit den/die Wirkstoff/e freigibt, **dadurch gekennzeichnet, dass**
- das Molekularsieb/Wirkstoff-Addukt (3, 7, 8) zusätzlich Kristallwasser enthält, und zwar derart, dass in Bezug auf eine ausreichende Grundmolmenge (m) an Kristallwasser das Molekularsieb partiell dehydratisiert ist, wobei das Molekularsieb mit der reduzierten Molmenge (m') an Kristallwasser mit dem/den Wirkstoff/en (Z, Z₁, Z₂, Z₃, Z₄) beladen ist, so dass bei Kontakt der Matrix (2, 6) mit dem Körpergewebe (4, 9) eine Adsorption von Wasser unter Desorption des entsprechenden Wirkstoffes stattfindet, wobei der Kristallwassergehalt des Molekularsiebes zunimmt.

2. Werkstoff nach Anspruch 1, **dadurch gekennzeichnet, dass** das Molekularsieb ein Metall-Aluminium-Silikat der folgenden Formel ist:
Meₙ [(AlO₂)_{X}· (SiO₂)_{Y}] · m(m')H₂O

3. Werkstoff nach Anspruch 2, **dadurch gekennzeichnet, dass** ein Metall der ersten oder zweiten Hauptgruppe des Periodensystems, vorzugsweise Natrium, Verwendung findet.

4. Werkstoff nach Anspruch 2 oder 3, **dadurch gekennzeichnet, dass** das Molekularsieb ein Natrium-Aluminium-Silikat der folgenden Formel ist:
Na₈₆ [(AlO₂)₈₆ · (SiO₂)₁₀₆] · m(m')H₂O

5. Werkstoff nach einem der Ansprüche 1 bis 4, **dadurch gekennzeichnet, dass** das Molekularsieb eine Grundmolmenge (m) an Kristallwasser von wenigstens 100, insbesondere 200, enthält.

6. Werkstoff nach Anspruch 5, **dadurch gekennzeichnet, dass** die Grundmolmenge (m) an Kristallwasser 276 beträgt.

7. Werkstoff nach Anspruch 4 und 6, **dadurch gekennzeichnet, dass** das Molekularsieb ein Natrium-Aluminium-Silikat der folgenden Formel ist:
Na₈₆ [(AlO₂)₈₆ · (SiO₂)₁₀₆] · 276 H₂O

8. Werkstoff nach einem der Ansprüche 1 bis 7, **dadurch gekennzeichnet, dass** das partiell dehydratisierte Molekularsieb einen Dehydratisierungsgrad von mindestens 20 %, vorzugsweise von 40 % bis 70 %, aufweist.

9. Werkstoff nach einem der Ansprüche 1 bis 8, **dadurch gekennzeichnet, dass** der Beladungsgrad des Wirkstoffes (Z, Z₁, Z₂, Z₃, Z₄) im Molekularsieb/Wirkstoff-Addukt (3, 7, 8) kleiner ist als der Dehydratisierungsgrad des partiell dehydratisierten Molekularsiebes.

10. Werkstoff nach Anspruch 9, **dadurch gekennzeichnet, dass** der Beladungsgrad mindestens 50 % des Dehydratisierungsgrades beträgt.

11. Werkstoff nach einem der Ansprüche 1 bis 10, **dadurch gekennzeichnet, dass** das Molekularsieb/Wirkstoff-Addukt (3, 7, 8) innerhalb der Matrix (2, 6) im wesentlichen gleichmäßig verteilt ist.

12. Werkstoff nach einem der Ansprüche 1 bis 11, **dadurch gekennzeichnet, dass** das Molekularsieb/Wirkstoff-Addukt (3, 7, 8) einen Anteil von 2 bis 30 Gew.-%, vorzugsweise 15 bis 20 Gew.-%, aufweist, und zwar bezogen auf die Gesamtmasse der Matrix (2, 6).

13. Werkstoff nach einem der Ansprüche 1 bis 12, **dadurch gekennzeichnet, dass** die Matrix (2, 6) ein Polymerwerkstoff, vorzugsweise ein Elastomer, ein thermoplastisches Elastomer oder ein thermoplastischer Kunststoff ist.

14. Werkstoff nach einem der Ansprüche 1 bis 13, **dadurch gekennzeichnet, dass** das Molekularsieb bei einem einheitlichen Dehydratisierungsgrad mit wenigstens einem ersten Wirkstoff (Z₁) mit fibrinolytischer Wirkung und einem zweiten Wirkstoff (Z₂) mit antibakterieller Wirkung beladen ist, die sich durch einen unterschiedlichen Beladungsgrad auszeichnen, so dass das Molekularsieb/Wirkstoff-Addukt (3) bei Kontakt der Matrix (2) mit dem Körpergewebe (4) die Wirkstoffe (Z₁, Z₂) sequentiell freigibt.

15. Werkstoff nach einem der Ansprüche 1 bis 13, **dadurch gekennzeichnet, dass** in die Matrix (6) wenigstens ein erstes und zweites Molekularsieb eingemischt sind, die typengleich sind, sich jedoch durch einen unterschiedlichen Dehydratisierungsgrad auszeichnen, wobei das erste Molekularsieb mit einem ersten Wirkstoff (Z₃) mit fibrinolytischer Wirkung und das zweite Molekularsieb mit einem zweiten Wirkstoff (Z₄) mit antibakterieller Wirkung beladen sind, und zwar bei etwa gleichem Beladungsgrad, so dass das erste Molekularsieb/Wirkstoff-Addukt (7) und das zweite Molekularsieb/Wirkstoff-Addukt (8) bei Kontakt der Matrix (6) mit dem Körpergewebe (9) die Wirkstoffe (Z₃, Z₄) sequentiell freigeben.

16. Werkstoff nach Anspruch 14 oder 15, **dadurch gekennzeichnet, dass** die sequentielle Freigabe der Wirkstoffe so erfolgt, dass der Wirkstoff (Z₁, Z₃) mit fibrinolytischer Wirkung schneller abgegeben wird als der Wirkstoff (Z₂, Z₄) mit antibakterieller Wirkung.

17. Verfahren zur Herstellung eines Werkstoffes nach einem der Ansprüche 1 bis 16, **gekennzeichnet durch** folgende Verfahrensschritte:
- das Molekularsieb mit einer ausreichenden Grundmolmenge (m) an Kristallwasser wird bei 300 bis 500°C, vorzugsweise bei 400 bis 450°C, mehrere Stunden, vorzugsweise 2 bis 6 Stunden, lang partiell dehydratisiert;
- anschließend wird das partiell dehydratisierte Molekularsieb mit der reduzierten Molmenge (m') an Kristallwasser mit dem/den Wirkstoff/en (Z, Z₁, Z₂, Z₃, Z₄) unter Bildung des entsprechenden Molekularsieb/Wirkstoff-Adduktes (3, 7, 8) beladen;
- schließlich wird das Molekularsieb/Wirkstoff-Addukt (3, 7, 8) in die Matrix (2, 6) eingemischt.

18. Verfahren zur Herstellung eines Werkstoffes nach Anspruch 17, **dadurch gekennzeichnet, dass** die Dehydratisierung und/oder Beladung in Gegenwart eines inerten Gases, beispielsweise von Stickstoff, durchgeführt wird/werden.

19. Verfahren zur Herstellung eines Werkstoffes nach Anspruch 17 oder 18, **dadurch gekennzeichnet, dass** die Dehydratisierung und/oder Beladung unter Normaldruck durchgeführt wird/werden.

## Claims

1. Material for manufacturing products (1, 5) having fibrinolytic and/or antibacterial properties, comprising at least one matrix (2, 6) which is compatible with body tissue and into which is mixed a molecular sieve which is charged with
- at least one active substance (Z) with a fibrinolytic or antibacterial action or
- at least a first active substance (Z₁, Z₃) having a fibrinolytic action and a second active substance (Z₂, Z₄) having an antibacterial action,
the corresponding molecular sieve/active substance adduct (3, 7, 8) releasing the active substance(s) when the matrix (2, 6) comes into contact with the body tissue (4, 9) in conjunction with the aqueous body fluid,
**characterised in that**
- the molecular sieve/active substance adduct (3, 7, 8) additionally contains water of crystallisation, such that the molecular sieve is partially dehydrated in terms of a sufficient basic molar amount (m) of water of crystallisation, the molecular sieve with the reduced molar amount (m') of water of crystallisation being charged with the active substance(s) (Z, Z₁, Z₂, Z₃, Z₄) so that when the matrix (2, 6) comes into contact with the body tissue (4, 9) water is adsorbed and the corresponding active substance is desorbed so that the content of water of crystallisation of the molecular sieve increases.

2. Material according to claim 1, **characterised in that** the molecular sieve is a metal aluminium silicate having the following formula:
Meₙ[(AlO₂)ₓ · (SiO₂)_{Y}] · m(m')H₂O

3. Material according to claim 2, **characterised in that** a metal of the first or second main group of the periodic table, preferably sodium, is used.

4. Material according to claim 2 or 3, **characterised in that** the molecular sieve is a sodium aluminium silicate having the following formula:
Na₈₆[(AlO₂)₈₆ · (SiO₂)₁₀₆] · m(m')H₂O

5. Material according to one of claims 1 to 4, **characterised in that** the molecular sieve contains a basic molar amount (m) of water of crystallisation of at least 100, in particular 200.

6. Material according to claim 5, **characterised in that** the basic molar amount (m) of water of crystallisation is 276.

7. Material according to claims 4 and 6, **characterised in that** the molecular sieve is a sodium aluminium silicate having the following formula:
Na₈₆[(AlO₂)₈₆ · (SiO₂)₁₀₆] · 276 H₂O

8. Material according to one of claims 1 to 7, **characterised in that** the partially dehydrated molecular sieve exhibits a degree of dehydration of at least 20%, preferably 40% to 70%.

9. Material according to one of claims 1 to 8, **characterised in that** the degree of charge of the active substance (Z, Z₁, Z₂, Z₃, Z₄) in the molecular sieve/active substance adduct (3, 7, 8) is less than the degree of dehydration of the partially dehydrated molecular sieve.

10. Material according to claim 9, **characterised in that** the degree of charge is at least 50% of the degree of dehydration.

11. Material according to one of claims 1 to 10, **characterised in that** the molecular sieve/active substance adduct (3, 7, 8) is distributed essentially uniformly within the matrix (2, 6).

12. Material according to one of claims 1 to 11, **characterised in that** the molecular sieve/active substance adduct (3, 7, 8) makes up 2 to 30% by weight, preferably 15 to 20% by weight, related to the total mass of the matrix (2, 6).

13. Material according to one of claims 1 to 12, **characterised in that** the matrix (2, 6) is a polymer material, preferably an elastomer, a thermoplastic elastomer or a thermoplastic plastic.

14. Material according to one of claims 1 to 13, **characterised in that** with a uniform degree of dehydration the molecular sieve is charged with at least a first active substance (Z₁) having a fibrinolytic action and a second active substance (Z₂) having an antibacterial action which are **characterised by** a different degree of charge so that the molecular sieve/active substance adduct (3) releases the active substances (Z₁, Z₂) sequentially when the matrix (2) comes into contact with the body tissue (4).

15. Material according to one of claims 1 to 13, **characterised in that** at least a first and a second molecular sieve of the same type but **characterised by** a different degree of dehydration are mixed into the matrix (6), the first molecular sieve being charged with a first active substance (Z₃) having a fibrinolytic action and the second molecular sieve being charged with a second active substance (Z₄) having an antibacterial action, with roughly the same degree of charge so that the first molecular sieve/active substance adduct (7) and the second molecular sieve/active substance adduct (8) release the active substances (Z₃, Z₄) sequentially when the matrix (6) comes into contact with the body tissue (9).

16. Material according to claim 14 or 15, **characterised in that** the active substances are released sequentially so that the active substance (Z₁, Z₃) having the fibrinolytic action is released more rapidly than the active substance (Z₂, Z₄) having the antibacterial action.

17. Method for manufacturing a material according to one of claims 1 to 16, **characterised by** the following steps:
- the molecular sieve with a sufficient basic molar amount (m) of water of crystallisation is partially dehydrated at 300 to 500°C, preferably 400 to 450°C, for a plurality of hours, preferably 2 to 6 hours;
- then the partially dehydrated molecular sieve with the reduced molar amount (m') of water of crystallisation is charged with the active substance(s) (Z, Z₁, Z₂, Z₃, Z₄) forming the corresponding molecular sieve/active substance adduct (3, 7, 8);
- finally, the molecular sieve/active substance adduct (3, 7, 8) is mixed into the matrix (2, 6).

18. Method for manufacturing a material according to claim 17, **characterised in that** the dehydration and/or charging is/are carried out in the presence of an inert gas, for example nitrogen.

19. Method for manufacturing a material according to claim 17 or 18, **characterised in that** the dehydration and/or charging is/are carried out at normal pressure.

## Revendications

1. Matériau de fabrication de produits (1, 5) dotés de propriétés fibrinolytiques et/ou antibactériennes, comprenant au moins une matrice (2,6) compatible avec les tissus corporels dans laquelle est intégré un tamis moléculaire qui est chargé avec
- au moins un principe actif (Z) exerçant un effet fibrinolytique ou antibactérien, ou
- au moins un premier principe actif (Z₁, Z₃) exerçant une action fibrinolytique et un deuxième principe actif (Z₂, Z₄) exerçant une action antibactérienne,
le produit d'addition tamis moléculaire/principe actif correspondant (3, 7, 8) libérant, par contact de la matrice (2, 6) avec le tissu corporel (4, 9) en relation avec le liquide corporel aqueux, le/les principe(s) actif(s), **caractérisé en ce que**
- le produit d'addition tamis moléculaire/principe actif (3, 7, 8) contient en outre de l'eau de cristallisation et cela, de telle sorte que, par rapport à une quantité molaire de base suffisante (m) d'eau de cristallisation, le tamis moléculaire est partiellement déshydraté, le tamis moléculaire présentant la quantité molaire réduite (m') d'eau de cristallisation étant chargé avec le/les principe(s) actif(s) (Z, Z₁, Z₂, Z₃, Z₄) de sorte que, par contact de la matrice (2, 6) avec le tissu corporel (4, 9), une adsorption d'eau s'effectue par désorption du principe actif correspondant, la teneur en eau de cristallisation du tamis moléculaire augmentant.

2. Matériau selon la revendication 1, **caractérisé en ce que** le tamis moléculaire est un silicate de métal-aluminium de formule suivante :
Meₙ[(AlO₂)ₓ · (SiO₂)_{y}] · m(m')H₂O

3. Matériau selon la revendication 2, **caractérisé en ce qu'**un métal du premier ou du deuxième groupe principal du système périodique, de préférence le sodium, est utilisé.

4. Matériau selon la revendication 2 ou 3, **caractérisé en ce que** le tamis moléculaire est un silicate de sodium-aluminium de formule suivante :
Na₈₆[(AlO₂)₈₆ · (SiO₂)₁₀₆] · m(m')H₂O

5. Matériau selon l'une quelconque des revendications 1 à 4, **caractérisé en ce que** le tamis moléculaire contient une quantité molaire de base (m) d'eau de cristallisation d'au moins 100, en particulier 200.

6. Matériau selon la revendication 5, **caractérisé en ce que** la quantité molaire de base (m) d'eau de cristallisation est de 276.

7. Matériau selon les revendications 4 et 6, **caractérisé en ce que** le tamis moléculaire est un silicate de sodium-aluminium de formule suivante :
Na₈₆[(AlO₂)₈₆ · (SiO₂)₁₀₆] · 276 H₂O

8. Matériau selon l'une quelconque des revendications 1 à 7, **caractérisé en ce que** le tamis moléculaire partiellement déshydraté présente un degré de déshydratation d'au moins 20 %, de préférence de 40 % à 70 %.

9. Matériau selon l'une quelconque des revendications 1 à 8, **caractérisé en ce que** le degré de charge du matériau (Z, Z₁, Z₂, Z₃, Z₄) dans le produit d'addition tamis moléculaire/principe actif (3, 7, 8) est inférieur au degré de déshydratation du tamis moléculaire partiellement déshydraté.

10. Matériau selon la revendication 9, **caractérisé en ce que** le degré de charge est d'au moins 50 % de celui du degré de déshydratation.

11. Matériau selon l'une quelconque des revendications 1 à 10, **caractérisé en ce que** le produit d'addition tamis moléculaire/principe actif (3, 7, 8) est réparti au sein de la matrice (2, 6) de manière essentiellement uniforme.

12. Matériau selon l'une quelconque des revendications 1 à 11, **caractérisé en ce que** le produit d'addition tamis moléculaire/principe actif (3, 7, 8) présente une proportion de 2 à 30 % en poids, de préférence de 15 à 20 % en poids par rapport à la masse totale de la matrice (2, 6).

13. Matériau selon l'une quelconque des revendications 1 à 12, **caractérisé en ce que** la matrice (2, 6) est un matériau polymère, de préférence un élastomère, un élastomère thermoplastique ou une matière synthétique thermoplastique.

14. Matériau selon l'une quelconque des revendications 1 à 13, **caractérisé en ce que** le tamis moléculaire présentant un degré de déshydratation unitaire est chargé avec au moins un premier principe actif (Z₁) exerçant une action fibrinolytique et un deuxième principe actif (Z₂) exerçant une action antibactérienne qui se caractérisent par un degré de charge différent, de sorte que le produit d'addition tamis moléculaire/principe actif (3) libère séquentiellement les principes actifs (Z₁, Z₂) par contact de la matrice (2) avec le tissu corporel (4).

15. Matériau selon l'une quelconque des revendications 1 à 13, **caractérisé en ce que**, dans la matrice (6), au moins un premier et un deuxième tamis moléculaires sont intégrés, qui sont de même type mais qui se caractérisent toutefois par un degré de déshydratation différent, le premier tamis moléculaire étant chargé d'un premier principe actif (Z₃) exerçant une action fibrinolytique et le deuxième tamis moléculaire étant chargé d'un deuxième principe actif (Z₄) exerçant une action antibactérienne et cela, avec un degré de charge à peu près identique, de sorte que le premier produit d'addition tamis moléculaire/principe actif (7) et le deuxième produit d'addition tamis moléculaire/principe actif (8) libèrent séquentiellement les principes actifs (Z₃, Z₄) par contact de la matrice (6) avec les tissus corporels (9).

16. Matériau selon la revendication 14 ou 15, **caractérisé en ce que** la libération séquentielle des principes actifs s'effectue de telle sorte que le principe actif (Z₁, Z₃) exerçant une action fibrinolytique est libéré plus rapidement que le principe actif (Z₂, Z₄) exerçant une action antibactérienne.

17. Procédé de fabrication d'un matériau selon l'une quelconque des revendications 1 à 16, **caractérisé par** les étapes suivantes du procédé :
- le tamis moléculaire présentant une quantité molaire de base suffisante (m) d'eau de cristallisation est partiellement déshydraté de 300 à 500° C, de préférence de 400 à 450° C, pendant plusieurs heures, de préférence, de 2 à 6 heures ;
- ensuite, le tamis moléculaire partiellement déshydraté présentant la quantité molaire réduite (m') d'eau de cristallisation est chargé avec le/les principe(s) actif(s) (Z, Z₁, Z₂, Z₃, Z₄) en formant le produit d'addition tamis moléculaire/principe actif correspondant (3, 7, 8) ;
- enfin, le produit d'addition tamis moléculaire/principe actif (3, 7, 8) est intégré dans la matrice (2, 6).

18. Procédé de fabrication d'un matériau selon la revendication 17, **caractérisé en ce que** la déshydratation et/ou le chargement s'effectue(nt) en présence d'un gaz inerte, par exemple de l'azote.

19. Procédé de fabrication d'un principe actif selon la revendication 17 ou 18, **caractérisé en ce que** le déshydratation et/ou le chargement s'effectue (nt) sous pression normale.
